# EUROPEAN PATENT SPECIFICATION

(11) **EP 3 037 044 B1**
(45) Date of publication and mention of the grant of the patent: **17.07.2019**
(21) Application number: 14199686.8
(22) Date of filing: 22.12.2014
(51) Int. Cl.: A61B 17/02

(54) **Medical instrument kit for manipulating, in particular retracting tissue or an organ**
Medizinisches Intrumentenset zur Manipulation insbesondere zur Retraktion von Gewebe oder eines Organs
Set d'instrument médical pour la manipulation, notamment pour le retrait de tissu ou d'organe

(43) Date of publication of application: 29.06.2016
(73) Proprietor: University Of Dundee, Dundee DD1 4HN (GB)
(72) Inventor: Wang, Zhigang, Dundee Medipark, Dundee DD2 1FD (GB); Cuschieri, Alfred, St. Andrews Fife KY16 9TY (GB)
(74) Representative: Witte, Weller & Partner Patentanwälte mbB

(56) References cited:
- US-A1- 2007 135 685
- US-A1- 2009 192 344
- US-A1- 2009 306 686
- US-B1- 7 338 434

## Description

The invention relates to a medical instrument kit for manipulating, in particular retracting tissue or an organ in the human or animal body.

In surgical operations on patients, it is often necessary to manipulate tissue or organs during the execution of surgical operations. Such manipulations can consist in displacing aside an organ or tissue in order to expose tissue or organs lying deeper on which the surgical task will be performed, or in retracting tissue or an organ for a subsequent dissection. In this sense, manipulating can involve any kind of moving, turning, displacing and in particular retracting tissue or organs.

Surgical tasks necessitating the manipulation of tissue or organs are an essential part of minimally invasive as well as open operations.

Conventional instruments used for manipulating tissue employ grasping jaws at the distal end portion of the instrument, which are operated from the handle at the proximal end of the instrument. The jaws are opened and then closed on the tissue exerting a grasping action on the tissue in order to hold and hence manipulate same. Such conventional grasping instruments have several disadvantages, because in grasping the tissues the jaws can cause direct trauma to the surface and internal structures of the tissue/organ. Furthermore, since such pressures may occlude the vascular supply, the tissues may be rendered ischemic.

The use of magnetic forces has been conceived as a solution to overcome these drawbacks of conventional graspers in manipulating tissues or organs. US 2007/0135685 A1 discloses a medical instrument for manipulating, including retracting tissue or an organ in the human or animal body. This comprises an elongated shaft and at least one working element placed at the distal end of the shaft, the working element comprising at least one magnetically acting element generating a magnetic field for manipulating the tissue or organ. For manipulation by the magnetic instrument, the tissue or organ is treated with a magnetically acting substance, for example by applying a small volume of glue-based magnetic media to the tissue surface or by interstitial injection of fluids, for example ferro-fluids which are magnetized in a magnetic field and hence attracted by the magnetically acting element of the working element through magnetic forces.

Further documents relating to instruments and methods for magnetic manipulation of tissue and organs are US 2007/0270629 A1, US 2007/0135679 A1, US 2007/0108860 A1, US 2008/0171907 A1, US 2010/0204727 A1, and US 2010/0168523 A1.

The use of magnetic forces for manipulating tissue has the main advantage that the manipulation can be carried out with reduced contact pressure between the tissue and the instrument thus avoiding or at least reducing trauma to the tissue or organ.

However, tissue magnetization by treating the tissue or organ to be manipulated with a magnetically acting substance has some disadvantages. One disadvantage is a weak magnetic attraction force due to the tissue gap between the injected magnetic materials, for example particles, and the magnetically acting element of the instrument. The second disadvantage of injection magnetization is that it is difficult to completely remove the injected magnetic particles after the surgery, particularly if the particles are larger than nanometer size to generate sufficient magnetic response. Hence, extra procedures are needed to remove completely the injected material. The third disadvantage is that not all organs or tissues are suitable for injection magnetization, and, tissue damage may be sustained by the needle injection.

Nowadays, muco-adhesive polymers are available which hydrate on contact with moist tissues and adhere to organs or tissues by virtue of ionic and hydrogen bonding as well as other interfacial forces, e.g. van der Waals interactions, lipid penetration etc. However, until recently the use of muco-adhesive polymers has been largely restricted to drug delivery, especially for controlled release of drugs and hormones.

US 2007/0135685 A1 mentioned above discloses a medical instrument for manipulating, in particular retracting tissue or an organ in the human or animal body, comprising an elongated shaft having a distal portion introducible into the body, and at least one working element arranged at a distal end portion of the shaft for manipulating the tissue or organ. The at least one working element comprises at least one magnetically acting element producing a magnetic field for manipulating the tissue or organ.

US 2009/0192344 A1 discloses a surgical kit which can be used to manipulate tissue within the body of the patient to create a working space within the body to allow a surgeon to easily access and work within the body using various surgical instruments. The surgical kit can include an implant comprised of a magnetic material which can be engaged with tissue within the body. The kit can further include a surgical instrument having a magnet which can be used to manipulate the implant and tissue engaged therewith.

US 7,338,434 B1 discloses a heart positioning device and system for positioning, manipulating, holding, grasping, immobilizing and/or stabilizing a heart. The heart positioning device may include a suction head and a shaft with a means for remotely changing the position of the head from a first position axially aligned with the shaft to a second, unaligned position and a sleeve slideably positioned on the shaft and sized to receive the suction head in a compressed condition.

US 2009/0306686 A1 discloses a medical holding apparatus provided with an anchor member composed of a tying-up portion to be tied up to an intracorporeal tissue and a first connection portion, and a locking member composed of a holding portion for holding another intracorporeal tissue, a medical instrument or a drug and a second connection portion, in which the first connection portion and the second connection portion can be detachably connected to each other.

In view of the disadvantages of prior art medical systems for manipulating tissue or an organ in the human or animal body based on magnetization of the tissue or organ itself, it is an object of the present invention to provide a medical instrument kit for manipulating, in particular retracting tissue or an organ, which dispenses with the need for magnetization of the tissue or organ itself. Additionally, the medical instrument kit described is suited for use in minimally invasive or minimal access surgery.

According to the invention, a medical instrument kit for manipulating, in particular retracting tissue or an organ in the human or animal body is provided, comprising: a muco-adhesive element having a flat-spread muco-adhesive polymer body configured to adhere to the surface of the tissue or the organ, wherein the muco-adhesive element further has at least one magnetically acting portion; an applicator configured to receive the muco-adhesive element and to introduce the muco-adhesive element into the body and for placing the muco-adhesive element on the tissue or organ to be manipulated, wherein the applicator is configured to be introduced via a minimal access port in the body surface into the body; and a manipulator configured to magnetically couple to the at least one magnetically acting portion of the muco-adhesive element, so that the tissue or organ to which the muco-adhesive element adheres can be manipulated through magnetic coupling between the manipulator and the at least one magnetically acting portion of the muco-adhesive element.

The medical instrument kit according to the invention makes use of a muco-adhesive element which itself has at least one magnetically acting portion. Thus, when using the medical instrument kit according to the invention, it is no longer necessary to magnetize the tissue or organ. The magnetic interaction or magnetic coupling for manipulating the tissue or organ occurs between the manipulator and the muco-adhesive element which in turn adheres to the tissue or organ. The muco-adhesive element has a flat-spread muco-adhesive polymer body configured to adhere to the surface of the tissue or the organ by virtue of ionic and hydrogen bonding as well as other interfacial forces, e.g. van der Waals interactions, lipid penetration etc. The magnetically acting portion can have one or more permanent magnets, or one or more elements which can be made of a material which can be magnetized in a magnetic field, e.g. ferromagnetic materials such as stainless steel type 410 or 430, or superparamagnetic iron oxide nanoparticles. Preferably, the at least one magnetically acting portion is arranged in the middle or central portion of the polymer body.

Further according to the invention, the medical instrument kit comprises an applicator configured to receive the muco-adhesive element and to introduce the muco-adhesive element into the body and to place the muco-adhesive element on the tissue or organ to be manipulated. The applicator is configured to be introduced through a minimal access port through the body surface/wall into the appropriate body cavity, whereby the medical instrument kit according to the invention is particularly suited for minimally invasive or minimal access surgery.

The muco-adhesive polymer flat body can have a two-dimensional area covering a surface of the tissue or organ to be manipulated as large as several square centimeters, depending on the size of the tissue or organ to be manipulated, thus providing a good and secure muco-adherence to the surface of the tissue or organ. Retraction forces before detachment of the muco-adhesive element from the tissue or organ of several Newton can be achieved which are sufficient for reliable manipulation and retraction of the tissue or organ.

However, it is to be understood that the medical instrument kit according to the invention can comprise a plurality of muco-adhesive elements which can be sequentially deployed via the same access port or via different access ports and adhered to different areas of the target tissue/organ. This can have the advantage of increasing magnetic coupling force, which would be more helpful in extra-corporal remote magnetic coupling where a much larger magnet or magnet system can be used externally, and the advantage of a smaller diameter port, such as a 5 mm port, because the muco-adhesive elements can be made smaller.

The applicator and the manipulator are combined as one instrument.

This configuration is particularly advantageous because the number of parts of the medical instrument kit can be kept low, and handling of the medical instrument kit for applying the muco-adhesive element and manipulating same is facilitated. In particular, the manipulator can be used to insert the muco-adhesive element into the applicator, before the surgery outside the patient's body as well as inside the patient's body while the surgery is performed. Removal of the muco-adhesive element from the body using the combined applicator/manipulator is thus facilitated.

In a preferred configuration, the muco-adhesive polymer body is flexible so that it is foldable and/or can be rolled or wrapped for easy insertion into the applicator.

In this configuration, if the polymer body of the muco-adhesive element is flexible or soft, in particular elastic, the muco-adhesive element can be brought into a geometrical configuration which is small enough for accommodation in the applicator, the latter for example configured as a sleeve.

The flexibility of the polymer body is improved if, as provided in a further configuration, the polymer body is a thin film preferably having a thickness of less than 1 mm.

The advantage of a polymer film with a thickness in the before-mentioned range is that the muco-adhesive element can be folded or rolled or wrapped into to a very small configuration requiring only a very little space, so that the applicator can be configured with a small diameter.

A further improvement of the muco-adhesive element is achieved in this regard, if the polymer body has a plurality of strips, which can be synonymously referred to as leaves, wherein first longitudinal ends of the strips are connected with one another to form a central portion of the polymer body, and wherein second longitudinal ends of the strips opposite to the first longitudinal ends are free ends, wherein the strips extend in radial direction from the central portion.

For inserting the muco-adhesive element into the applicator, the strips can be easily folded into a slim elongated shape in this refinement. If the applicator is configured as a tube or sleeve, the strips fold or bend themselves upon drawing the muco-adhesive element into the applicator. Further, unfolding the strips at the surgical site can be achieved automatically upon pushing the muco-adhesive element out of the applicator, if the polymer body is flexible, and in particular elastic. If necessary, additional methods can be used to assist unfolding the flexible/elastic polymer strips, such as using another instrument, which can be inserted via another minimal access port to assist opening and placing the muco-adhesive polymer element, or using an inflatable balloon to radially expand the strips once they are pushed out of the applicator.

Preferably, each strip has a two-dimensional shape which widens from the first longitudinal end to the free end.

The number of strips preferably ranges from two to eight strips. In the configuration of the muco-adhesive element having a plurality of strips, hence the muco-adhesive element resembles the shape of a star.

In a further preferred configuration, the polymer body has at least partially a stiffening layer for increasing the mechanical strength of the polymer body.

This configuration is particularly advantageous in connection with the before-mentioned configuration according to which the polymer body is a thin polymer film. In particular in the region of the at least one magnetically active portion, the magnetic attraction forces between the manipulator and the muco-adhesive element and thus the tensile forces on the muco-adhesive element are highest during manipulating the tissue or organ, so that the stiffening layer advantageously is provided in this region of higher tensile forces to increase the mechanical strength of the muco-adhesive element in this area.

In a further preferred configuration, the at least one magnetically acting portion of the muco-adhesive element has at least one magnet fixed to the polymer body.

Although it is conceivable to provide the at least one magnetically acting portion by adding magnetizable or magnetized particles to the polymer of the polymer body, this configuration is advantageous in reducing the manufacturing cost of the muco-adhesive element. In particular, the at least one magnet can be reused with another polymer body. The at least one magnet can be a permanent magnet or magnetic stainless steel.

Preferably, the at least one magnet is fixed to the polymer body in the middle or central portion of the polymer body, in particular if the muco-adhesive element is configured in star-shape as mentioned before. Medical grade cyanoacrylate glue such as Loctite 4014 can be used for such fixation.

Further preferably, the at least one magnet comprises two magnets, wherein the polymer body is sandwiched between the two magnets.

This configuration has the advantage that when the two magnets are permanent magnets or one of the two magnets is a permanent magnet and the other is magnetic stainless steel, they can fix themselves to the polymer body by magnetic attraction between the two magnets so that fixing the magnets to the polymer body is facilitated. Other fixing means like gluing, which could be conceived as well for fixing the at least one magnet to the polymer body can be principally dispensed with in this configuration.

In connection with the above-mentioned configuration of a star-shaped design of the muco-adhesive element comprising a plurality of strips, it is further preferred if the two magnets connect the first ends of the strips to one another by a clamping force produced by mutual magnetic attraction between the two magnets.

This configuration is particularly advantageous, because the two magnets do not only fix themselves to the polymer body of the muco-adhesive element, but they also connect and fix the first longitudinal ends of the strips to one another. In this configuration, the muco-adhesive element can be advantageously built up from a plurality of individual muco-adhesive strips which are then connected to one another by means of the two magnets.

In a further preferred configuration, the two magnets are ring magnets defining a through hole for receiving a further magnet.

While the two ring magnets predominantly can serve to connect and fix the first longitudinal ends of the strips to one another in the star-shaped configuration of the muco-adhesive element, the further magnet can increase the magnetic response when interacting with the manipulator, in particular if the manipulator couples to the at least one magnetically acting portion of the muco-adhesive element from the exterior of the body of the patient.

In a further preferred configuration, the applicator has an elongated outer tube for receiving the muco-adhesive element, and an elongated slider on the outer tube.

In this configuration, the applicator can be manufactured with low cost. Further, applying the muco-adhesive element with the applicator in this configuration can be maneuvered very easily by introducing the applicator with the muco-adhesive element inserted therein through a minimal access port into the body of the patient, and by pushing the slider in distal direction, the muco-adhesive element is pushed out of the outer tube of the applicator.

It is further preferred, if the slider is a second tube, and wherein the manipulator is configured as a probe adapted to slide in the second tube and having a distal magnetic tip for magnetic coupling to the at least one magnetically acting portion of the muco-adhesive element. The distal magnetic tip can be a permanent magnet or electromagnet.

In this configuration of the combined applicator/manipulator, all the functions of applying, manipulating and withdrawing the muco-adhesive element can be fulfilled with only a three-part design of the applicator/manipulator thus only requiring a low number of parts. The muco-adhesive element can be drawn into the outer tube of the applicator by means of the probe so that no further instrument is required to insert the muco-adhesive element into the outer tube. The same probe which remains in the second tube is used for manipulating the muco-adhesive element applied to the tissue or organ in the patient's body. Further the probe is easily guided in the second tube thus facilitating the manipulating, in particular retracting of the tissue or organ. When the distal magnetic tip is an electromagnet, an external power generator is used to provide electrical current to the electromagnet to switch on and off the electromagnetic field and to change the magnetic field strength by changing the amplitude of the electrical current.

It is further preferred, if the second tube has a distal non-magnetic stop.

The non-magnetic stop at the distal end of the second tube of the applicator has the advantage, that the magnetic tip of the probe can be easily detached from the muco-adhesive element by withdrawing the probe, while the magnetically acting portion of the muco-adhesive element is stopped by the non-magnetic stop. Thus, the manipulator/applicator can be easily detached from the muco-adhesive element at the surgical site in the patient's body without requiring additional instrument for this procedure. The non-magnetic stop on the other hand does not shield the magnetic field between the magnetically acting portion of the muco-adhesive element and the magnetic tip of the probe thus providing a reliable attraction force between them to ensure reliable retraction of the tissue or organ when withdrawing the probe together with the second tube for manipulating the tissue or organ.

When an electromagnet is used as the distal magnetic tip of the manipulator, the second tube and its distal non-magnetic stop are not needed.

In further preferred configurations the muco-adhesive element and/or the further magnet and/or the probe and/or the non-magnetic stop has/have a central through hole extending in longitudinal direction for delivering a fluid for moistening the tissue or organ.

This configuration has the advantage that fluid delivery can be easily accomplished through the applicator/manipulator without needing a second access port. Fluid delivery can be helpful, in order to keep the tissue or organ sufficiently moist for a good adherence of the polymer body of the muco-adhesive element to the tissue or organ. On the other hand, by delivering a sufficient amount of fluid to the tissue or organ, the muco-adhesive element can be detached from the tissue or organ after the surgery in a controlled and easy manner.

The muco-adhesive polymer body preferably is made of a chemical formulation comprising 30% carbopol 971, 30% poloxamer, 20% hydroxypropyl methylcellulose (HPMC), 10% polyvinyl pyrrolidone (PVP), and 10% propylene glycol (PG).

In another configuration, the muco-adhesive polymer body is made of a chemical formulation comprising 45% carbopol 971, 10% poloxamer 407, and 45% HPMC.

Further features and advantages emerge from the following description and the accompanying drawings.

It is to be understood, that the before-mentioned features and those still to be described below are not only applicable in the combinations given, but also in other combinations and in isolation without departing from the scope of the present invention.

Exemplary embodiments are shown in the drawings and will be described hereinafter with reference thereto. In the drawings:
- Fig. 1: shows an embodiment of a medical instrument kit for manipulating, in particular retracting tissue or an organ in the human or animal body;
- Fig. 2: shows an embodiment of a muco-adhesive element of the instrument kit in Fig. 1 in a perspective view;
- Fig. 3: shows a detail of the muco-adhesive element in Fig. 2 in a perspective view;
- Fig. 4: shows another detail of the muco-adhesive element in Fig. 2 in a perspective view;
- Fig. 5: shows a further detail of the muco-adhesive element in Fig. 2 in a perspective view;
- Fig. 6: shows an embodiment of an applicator and manipulator of the instrument kit in Fig. 1 in a disassembled state and in a perspective side view;
- Fig. 6A: shows an additional component of the applicator in Fig. 6 in two operation states;
- Fig. 7: shows the applicator and manipulator in Fig. 6 in an assembled state in a perspective side view;
- Fig. 8, 9 and 10: show single steps of loading the applicator with the muco-adhesive element in perspective side views;
- Fig. 11: shows introducing the loaded applicator through a minimal access port through the body surface of a patient's body;
- Fig. 11A: shows pushing out the muco-adhesive element from the applicator in a state departing from Fig. 11, wherein the body surface and tissue shown in Fig. 11 are omitted in Fig. 11A;
- Fig. 11B: shows un-folding the muco-adhesive element using the component in Fig. 6A;
- Fig. 12: shows the muco-adhesive element adhering to tissue before retracting the tissue with the aid of the manipulator;
- Fig. 13: shows a retracted state of the tissue retracted by means of the manipulator;
- Fig. 14: shows the separation of the applicator and manipulator from the muco-adhesive element;
- Fig. 15: shows another embodiment of a manipulator for manipulating the tissue from outside the body surface of a patient's body;
- Fig. 16: shows removal of the muco-adhesive element from the tissue; and
- Fig. 17: shows the withdrawal of the medical instrument kit from a patient's body through the body surface.

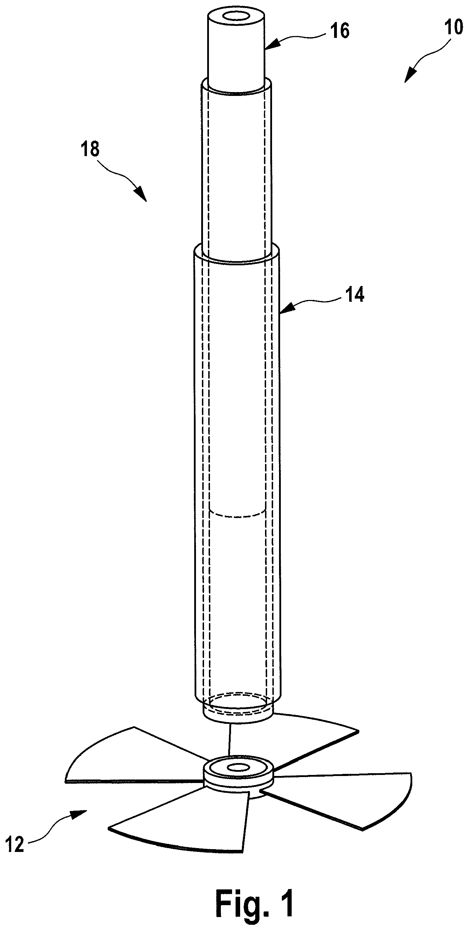

Fig. 1 shows an embodiment of a medical instrument kit 10 for manipulating, in particular retracting tissue or an organ in the human or animal body.

The instrument kit 10 comprises a muco-adhesive element 12, an applicator 14 and a manipulator 16. The applicator 14 and the manipulator 16 are combined with one another as one instrument 18 in this embodiment, which is also referred to as applicator/manipulator 18 herein.

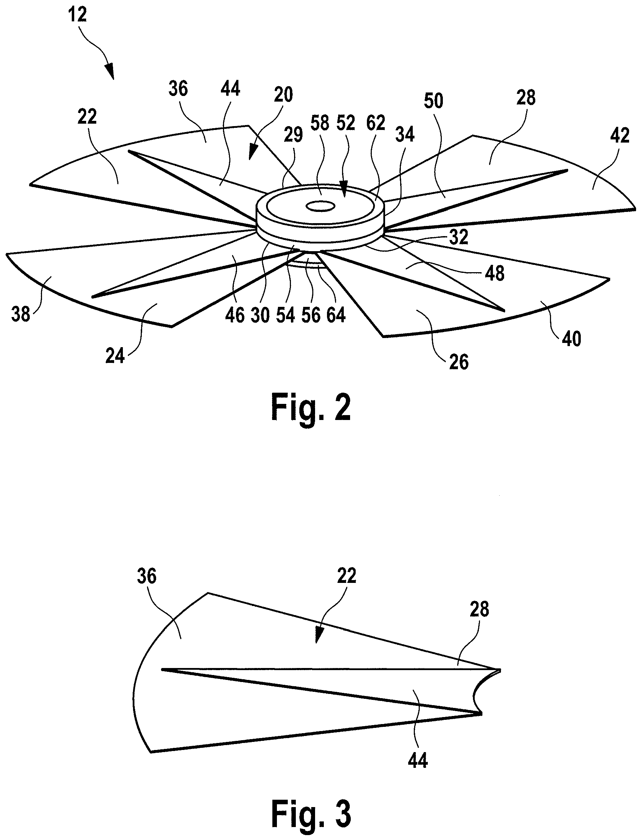

With reference to Figs. 2 through 5, the muco-adhesive element 12 will be described hereinafter.

The muco-adhesive element 12 has a flat-spread muco-adhesive polymer body 20. The polymer body 20 is configured to adhere to the tissue or organ to be manipulated. To this end, the muco-adhesive polymer body comprises a muco-adhesive polymer which hydrates on contact with moist tissues and adheres to organs or tissues by virtue of ionic and hydrogen bonding as well as other interfacial forces, for example, van der Waals interactions, lipid penetration.

In an embodiment, the muco-adhesive polymer comprises the following formulation: 30% carbopol 971, 30% poloxamer, 20% hydroxypropyl methylcellulose, 10% polyvinyl pyrrolidone; and 10% propylene glycol.

In another embodiment, the muco-adhesive polymer comprises the following formulation: 45% carbopol 971, 10% poloxamer 407, 45% hydroxypropyl methylcellulose.

As an example, to make the muco-adhesive polymer according to the formulation mentioned first, a mixture of carbopol 971 (450 mg), poloxamer (450 mg), hydroxypropyl methylcellulose (300 mg), polyvinyl pyrrolidone (150 mg) and propylene glycol (150 mg) are firstly added to deionized H₂O (90 mL), followed by the subsequent addition of triethanol amine (0.3 mL). The mixture is stirred at room temperature by an overhead mechanical stirrer at 300 rpm for 18 h. The resulting viscous gel is then centrifuged at 6000 to 8000 rpm for 15 min to remove trapped air bubbles. The clear viscous gel is transferred into a PTFE circular mold (diameter: 78 mm; height: 30 mm) and dried inside a fume hood at room temperature 4-5 days. Alternative drying methods can be carried out in an incubator at 30° C for 5-6 days. The film is completely dried for the ease of removal from PTFE mold and for storage.

In particular, the polymer body 20 is a polymer film having a thickness of less than 1 mm, wherein 0.1, 0.3, 0.5 mm thick films are suited example thicknesses of the polymer film.

The muco-adhesive polymer body 20 is soft or flexible, also due to its small thickness, so that the polymer body 20 is foldable or wrappable for inserting into the applicator 14 as will be described below in more detail.

Although the polymer body can have a full-plane design, the muco-adhesive polymer body 20 preferably has a plurality of strips (which can also be referred to as leaves) 22, 24, 26, 28. In the present embodiment, the muco-adhesive polymer body 20 has four strips 22, 24, 26, 28. Due to the design of the polymer body 20 having a plurality of strips, the polymer body 20 resembles the shape of a star.

It is to be understood, that the number of strips can be less than four or more than four, in particular in the range from two to eight strips.

Strip 22 has a first longitudinal end 29, strip 24 has a first longitudinal end 30, strip 26 has a first longitudinal end 32, and strip 28 has a first longitudinal end 34. The first longitudinal ends 29, 30, 32, 34 are connected with one another to form a central portion of the polymer body 20. First strip 22 has a second longitudinal end 36, strip 24 has a second longitudinal end 38, strip 26 has a second longitudinal end 40, and strip 28 has a second longitudinal end 42, wherein the second longitudinal ends 36, 38, 40, and 42 are free ends, i.e. the strips 22, 24, 26, and 28 are not connected to one another in the region of the second longitudinal ends 36, 38, 40, 42. The strips 22, 24, 26, and 28 extend in radial direction from the central portion, as shown in Fig. 2.

The strips 22, 24, 26, 28 widen in radial direction from the first longitudinal ends 29, 30, 32, 34 to the second longitudinal ends 36, 38, 40, 42.

In order to produce the star-shaped design of the polymer body 20, an initially full-plane muco-adhesive polymer film is provided, wherein segments are cut out from the full-plane sheet to form the strips 22, 24, 26, 28. In this case, the first longitudinal ends 29, 30, 32, 34 are connected to one another via remaining material bridges of the polymer film, which have not been cut away when cutting the polymer body 20 into the star-shaped design.

In another embodiment, the star-shaped design of the polymer body 20 can be produced in that the strips 22, 24, 26, 28 are provided as separate pre-produced parts, as, for example, shown for strip 22 in Fig. 3. In this case, the strips 22, 24, 26, 28 are subsequently connected to one another. This can be accomplished by any type of connection, for example by gluing. Another way to connect the first longitudinal ends 29, 30, 32, 34 with one another will be described below.

The polymer body 20 further has at least partially a stiffening layer for increasing the mechanical strength of the polymer body 20. In the embodiment shown in Fig. 2, strip 22 has a stiffening layer 44, strip 24 has a stiffening layer 46, strip 26 has a stiffening layer 48, and strip 28 has a stiffening layer 50. The stiffening layers 44, 46, 48, 50 are only provided over a part of each strip 22, 24, 26, 28. In particular, the coverage of the strips 22, 24, 26, 28 by the stiffening layers 44, 46, 48, 50 is larger in the region of the first longitudinal ends 29, 30, 32, 34 than in the remaining portion of the strips 22, 24, 26, 28.

The stiffening layers 44, 46, 48, 50 can be made of, for example, the same material as the remaining polymer body 20, or of a different material, e.g. silicone, and can have a thickness of less than 1 mm.

The muco-adhesive element 12 further has at least one magnetically acting portion, which is preferably arranged in the central portion of the polymer body 20.

In the present embodiment, the at least one magnetically acting portion 52 has at least one magnet fixed to the polymer body 20.

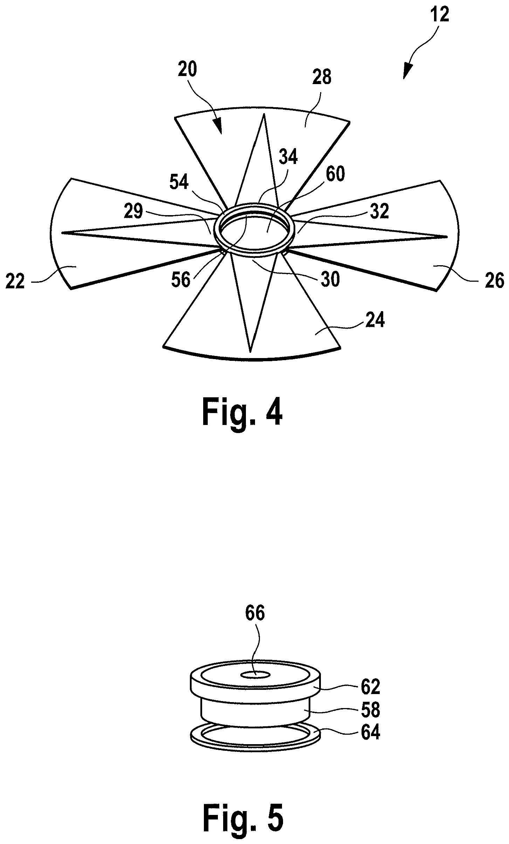

In more detail, the present embodiment of the magnetically acting portion 52 comprises two magnets 54 and 56 between which the polymer body 20 is sandwiched, as shown in Fig. 4. The magnets 54 and 56 are configured as ring magnets. The ring magnet 54 is arranged on a first side of the polymer body 20, and the ring magnet 56 is arranged on the opposite second side of the polymer body 20. In case that the star-shaped design of the polymer body 20 is produced by providing the strips 22, 24, 26, 28 as separate pieces or parts as shown for strip 22 in Fig. 3, the ring magnets 54 and 56 connect and fix the first longitudinal ends 29, 30, 32, 34 to one another by a clamping force produced by the mutual magnetic attraction of the two ring magnets 54, 56.

Instead of using two ring magnets, the magnets 54, 56 can also be disc-shaped magnets. In this case, the disc-shaped magnets preferably have a through hole in their center for fluid passage.

In the present case, where the two magnets 54 and 56 are ring magnets, the magnetically acting portion 52 has a further magnet 58 received in a through hole 60 of the magnets 54, 56 as shown in Fig. 4.

The trough hole 60 preferably also extends through the polymer body 20, i.e. there is no polymer material of the polymer body 20 in the through hole 60.

The further magnet 58 is shown in Fig. 5 in isolation. The arrangement further comprises to stop rings 62, 64 which fix the further magnet 58 to the ring magnets 54, 56, as shown in Fig. 2. In particular, the stop ring 64 can be configured such that the stop ring 64 enhances the clamping force for connecting the first longitudinal ends 29, 30, 32, 34 of the strips 22, 24, 26, 28 to one another.

As shown in Fig. 2 and 5, the further magnet 58 has a through hole 66 fully extending through the further magnet 58. The through hole 66 can be used for delivering a fluid to the underside of the polymer body 20, which underside is intended for adhering to the organ or tissue to be manipulated.

The further magnet 58 provides an additional magnetic force in addition to the magnetic force provided by the magnets 54, 56, for coupling to the manipulator 16 in a procedure of manipulating, in particular retracting tissue or an organ in a patient's body.

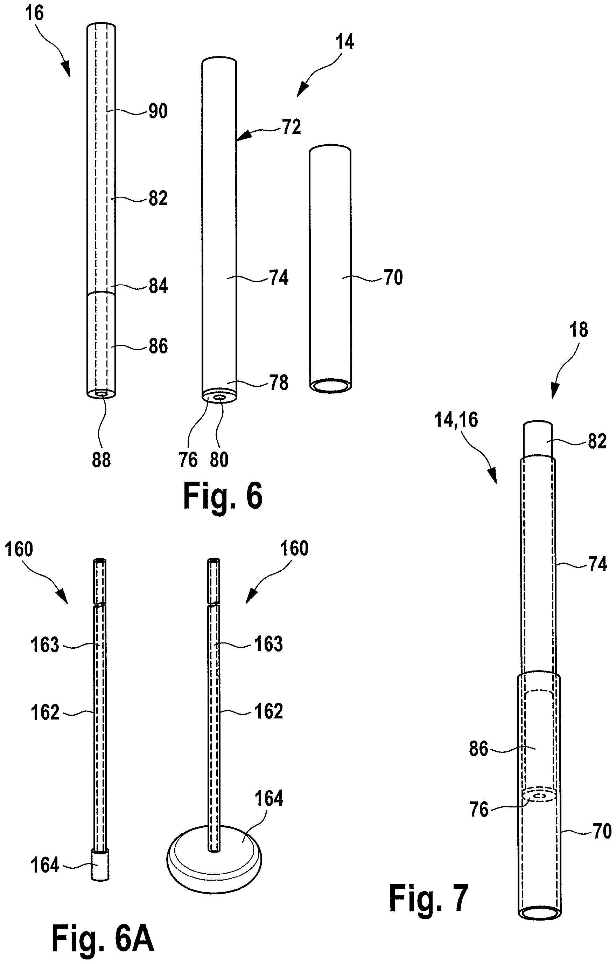

With reference to Fig. 6 and 7, the applicator 14 and the manipulator 16 will be described hereinafter.

Fig. 6 shows the applicator 14 and the manipulator 16 in a disassembled state. As already mentioned above, the applicator 14 and the manipulator 16 are combined to one instrument 18 according to Fig. 1, i.e. they are integrated into one another.

The applicator 14 has an elongated outer tube 70 and an elongated slider 72, which is adapted to slide in the outer tube 70, as shown in Fig. 7. The slider 72 is configured as a tube 74 having an outer diameter adapted to the inner diameter of the outer tube 70 so that the tube 74 can be inserted into the outer tube 70 and guided therein as shown in Fig. 7.

The slider 72 in form of the tube 74 has a distal non-magnetic stop 76 which can be configured as a disc arranged at the distal end 78 of the tube 74. The distal non-magnetic stop 76 has a through hole 80 for the passage of a fluid.

The manipulator 16 also has an elongated structure and comprises, in the present embodiment, a probe 82 having, at a distal end 84, a magnetic tip 86. The magnetic tip 86 can be configured as a tubular magnet meaning that the magnetic tip 86 has a through hole 88 fully extending through the magnetic tip 86 in longitudinal direction. This through hole 88 further communicates with a through hole 90 illustrated in Fig. 6 by broken lines fully extending through the remaining portion of the probe 82.

An outer diameter of the probe 82 including the magnetic tip 86 is adapted to an inner diameter of the tube 74 so that the manipulator 16 can be inserted into the tube 74 of the slider 72 and guided therein, as shown in Fig. 7. The slider 72 is slidable in the outer tube 70 relative thereto, and the manipulator 16 is slidable in the slider 72 relative thereto and also relative to the outer tube 70. This means that all three parts, namely the slider 72, the manipulator 16 and the outer tube 70 can be displaced in longitudinal direction independently of each other.

Fig. 6A shows an additional component 160 of the applicator 14 which can be used for unfolding the muco-adhesive element 12 after having been pushed out from the applicator 14, as will be described below. The additional component 160 has an elongated tube 162 having an outer diameter which is preferably smaller than the inner diameter of the through hole 90 of the probe 82 in Fig. 6. At the distal end of the tube 162, an inflatable member 164, for example a balloon, is arranged. The left part of Fig. 6A shows the deflated state of the inflatable member 164, and the right part of Fig. 6A shows the inflated state of the inflatable member 164. In the deflated state, the additional component 160 can be introduced through the trough hole 90 of the tube 82 until the inflatable member 164 protrudes from the distal end of the magnetic tip 86. Inflating the inflatable member 164 is performed through a through hole 163 in the tube 162 of the additional component 160. The additional component 160 is optional, and the applicator 14 can be used with or without the additional component 160.

The inflatable member 164 preferably has different material stiffness/elasticity at radial and axial directions, and when inflated, expands radially and forms a dish (or tire or wheel) as shown in the right part of Fig. 6A.

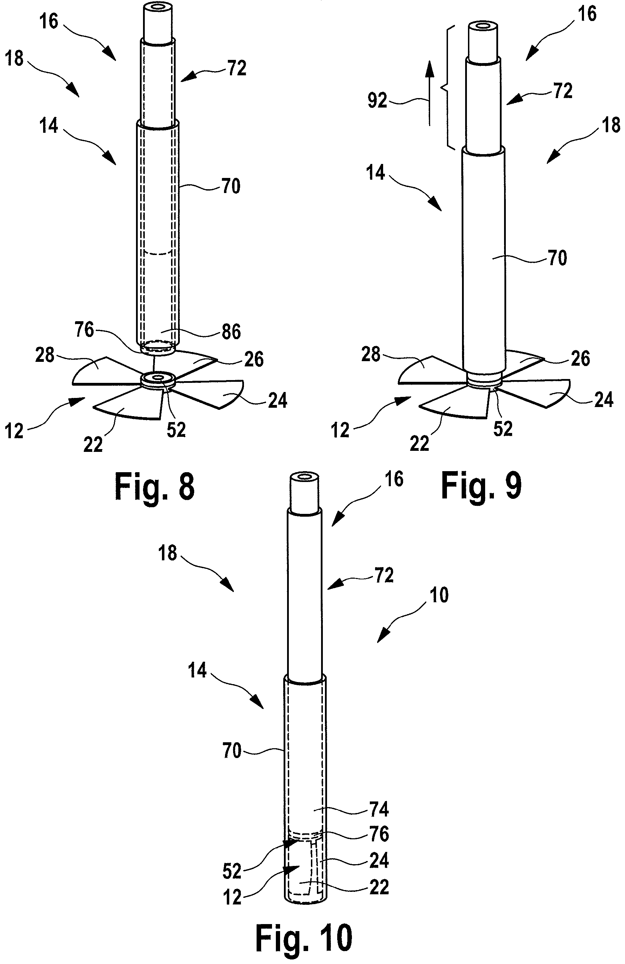

With reference to Fig. 8 through 10, loading of the applicator 14 with the muco-adhesive element 12 will be described hereinafter. Loading of the applicator 14 with the muco-adhesive element 12 is facilitated by also using the manipulator 16 in the loading procedure.

According to Fig. 8, the slider 72 inserted in the outer tube 70 is displaced relative to the outer tube 70 in distal direction so far that the non-magnetic stop 76 of the slider 72 extends beyond the distal end of the outer tube 70. The manipulator 16 inserted in the slider 72 is slided into the slider 72 so far that the magnetic tip 86 abutts against the non-magnetic stop 76. In this assembly state of the applicator 14, the magnetically acting portion 52 of the muco-adhesive element 12 is brought into contact with the non-magnetic stop 76 of the slider 72, as shown in Fig. 9. Due to the magnetic attraction forces acting between the magnetically acting portion 52 of the muco-adhesive element 12 and the magnetic tip 86 of the manipulator 16 (the attraction forces act through the non-magnetic stop 76), the muco-adhesive element 12 magnetically adheres to the non-magnetic stop 76.

Now, by retracting the manipulator 16 and the slider 72 together, without relative movement between them, in direction of an arrow 92 in Fig. 9, the muco-adhesive element 12 magnetically adhering to the stop 76 is drawn into the outer tube 70 until it is fully received in the outer tube 70 as shown in Fig. 10. It is to be understood that the magnetically acting portion 52 including the magnets 54, 56 and the stop rings 62, 64 has an outer diameter which fits into the inner diameter of the outer tube 70.

Upon drawing the muco-adhesive element 12 into the outer tube 70, the strips 22, 24, 26, 28 of the polymer body 20 of the muco-adhesive element 12 automatically fold in distal direction as shown in Fig. 10 for strips 22, 24.

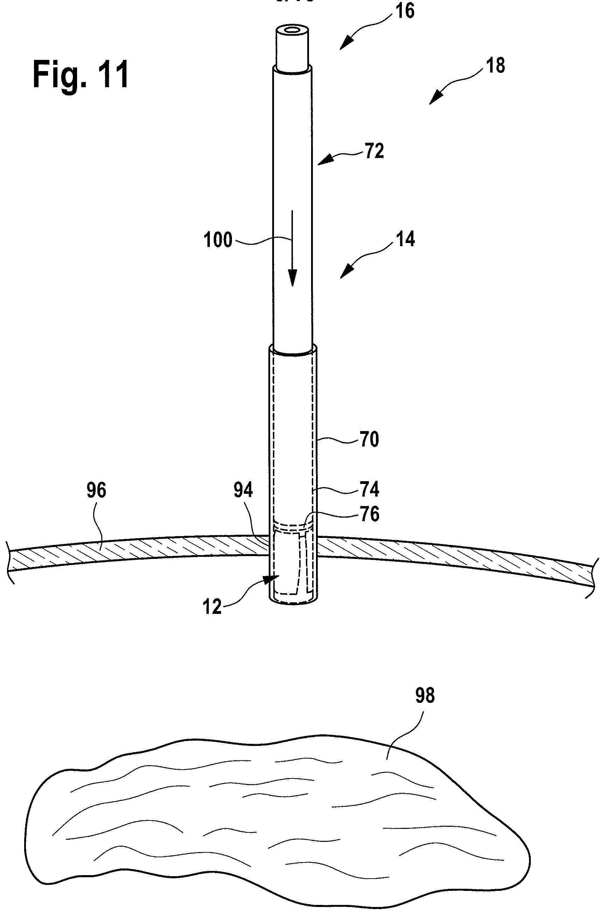

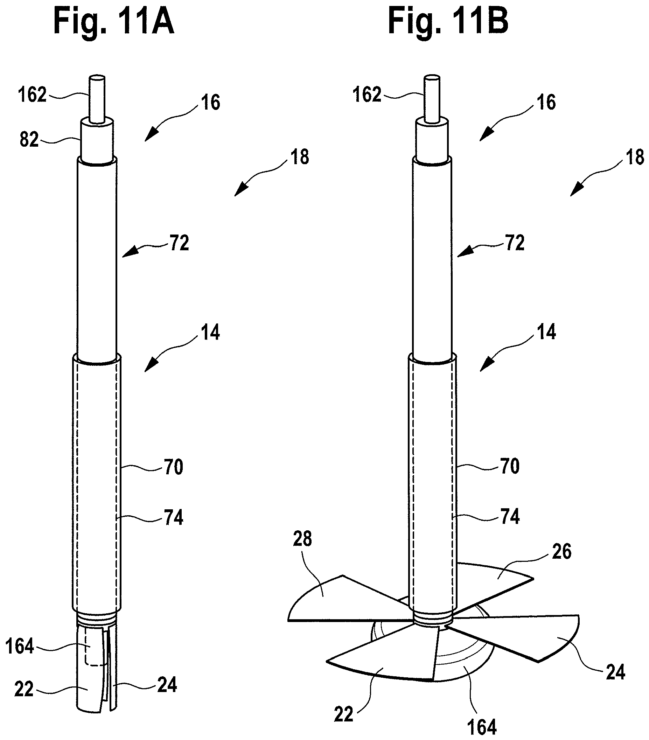

The medical instrument kit with the applicator 14 loaded with the muco-adhesive element 12 now is ready for introducing the muco-adhesive element 12 through a minimal access port 94 in the body surface 96 of a patient's body as shown in Fig. 11, and for placing the muco-adhesive element 12 on a tissue or organ 98 to be manipulated. After the outer tube 70 of the applicator 14 has been introduced through the minimal access port 94, the muco-adhesive element 12 can be pushed out of the outer tube 70 by pushing the slider 72 in distal direction according to an arrow 100 in Fig. 11. For pushing out the muco-adhesive element 12 from the outer tube 70, it is not necessary to also push the manipulator 16 in distal direction, because the non-magnetic stop 76 exerts the pushing force onto the muco-adhesive element 12. Nevertheless, in order to avoid an undesired premature detachment of the muco-adhesive element 12 from the applicator 14, it is advantageous to also push the manipulator 16 in distal direction when pushing out the muco-adhesive element 12 from the outer tube 70, in order to safely maintain the magnetic attraction between the magnetically acting portion 52 and the magnetic tip 86.

Upon pushing out the muco-adhesive element 12 from the outer tube 70, the strips 22, 24, 26, 28 of the polymer body 20 of the muco-adhesive element 12 spread or unfold at least to a certain degree automatically.

If the folded strips 22, 24, 26, 28 cannot unfold automatically to their flat-spread state prior to applying them to the surface of tissue 98 for adherence, this unfolding and placement onto the tissue 98 for adherence can be assisted by using another small instrument, such as a non-ferromagnetic grasper, which can be inserted via another access port (not shown). This additional instrument is to be used temporarily for assisting the unfolding and placing the strips 22, 24, 26, 28 onto the surface of the tissue 98. The additional instrument will be withdrawn from the second access port immediately. Hence, the second access port can be used for other instruments depending on the procedure requirement.

Further alternatively, and preferred, assistance/facilitation of the unfolding of the strips 22, 24, 26, 28 can be provided by the additional component 160 in fig. 6A, as shown in figs. 11A and 11B. The additional component 160 is introduced through the tube 82 of the manipulator 16 with the inflatable member 164 in the deflated state, until the latter emerges from the distal end of the applicator 14 as shown in fig. 11A. Then, the correctly located inflatable member 164 is inflated to expand radially, in order to push/unfold the folded polymer strips 22, 24, 26, 28 to their flat-spread state as shown in Fig. 11B. The inflatable member 164 is then deflated to its collapsed state and can be withdrawn from the applicator 14/manipulator 16, and the muco-adhesive element 12 is adhered to the surface of the tissue 98.

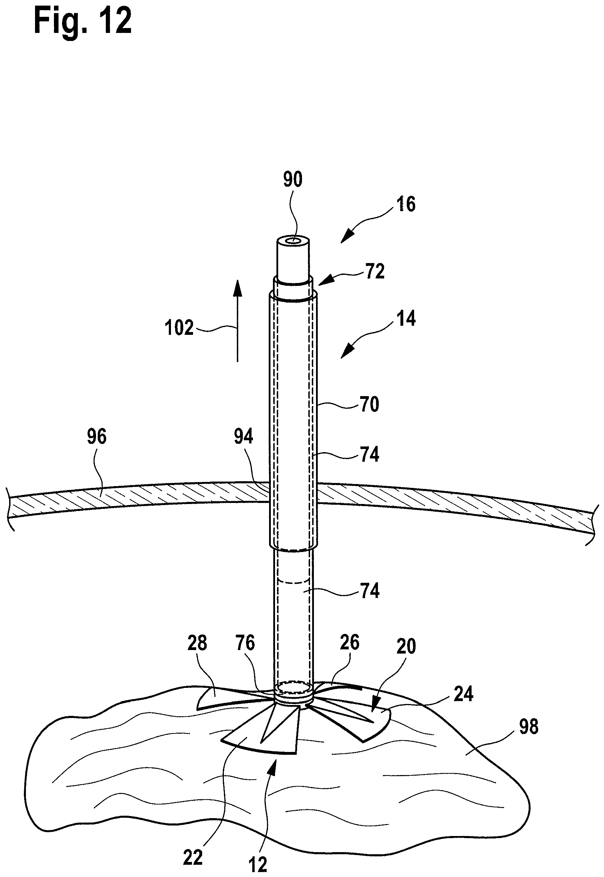

With reference to Fig. 12, by pushing the slider 72 relative to the outer tube 70 in distal direction, the muco-adhesive element 12 is placed onto the tissue 98 to be manipulated. As can be taken from a comparison between Fig. 11 and 12, the outer tube 70 of the applicator 14 has not been moved when pushing out the muco-adhesive element 12. In this respect, the outer tube 70 of the applicator 14 can be used as a trocar sleeve for guiding the slider 72.

Due to the flexible or soft nature of the polymer body 20, the polymer body 20 adapts itself to the surface topography of the tissue 98 as shown in Fig. 12.

Depending on the moisture of the surface of the tissue 98 and the time of contact between the polymer body 20 and the tissue 98, the polymer body 20 will adhere to the tissue 98 by virtue of its muco-adhesive properties. A contact time of less than one minute can be sufficient. In case that the surface of the tissue 98 is not sufficiently moist for a proper adherence of the polymer body 20 to the tissue 98, a small amount of fluid, like NaCI-solution, can be delivered through the through holes 90 and 88 of the manipulator 16 which then also passes through the through hole 80 of the non-magnetic stop 76 and the through hole 66 in the further magnet 58 and the through hole 60 in the polymer body 20 so that the fluid comes to the surface of the tissue 98 and the underside of the polymer body 20 which rests on the surface of the tissue 98.

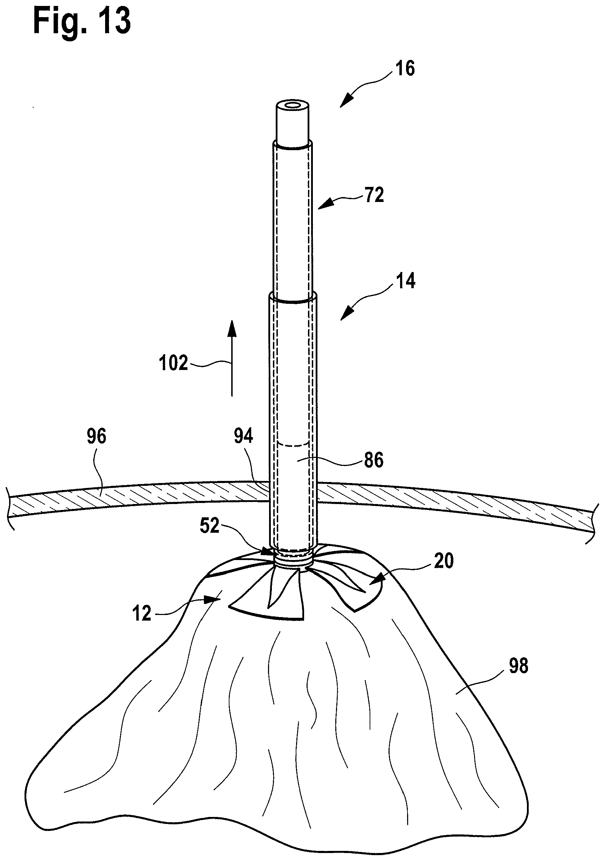

When the polymer body 20 of the muco-adhesive element 12 sufficiently adheres to the tissue 98, the tissue 98 can be manipulated, in particular retracted by retracting the slider 72 together with the manipulator 16 in proximal direction according to an arrow 102 in Fig. 12. Fig. 13 shows a retracted state of the tissue 98. The retraction of the tissue 98 is obtained as a magnetic retraction by virtue of the magnetic attractive forces acting between the magnetic tip 86 of the manipulator 16 and the magnetically acting portion 52 of the muco-adhesive element 12. The magnetic retraction forces exerted on the magnetically acting portion 52 of the muco-adhesive element 12 are transferred from the muco-adhesive element 12 to the tissue 98, by virtue of the muco-adherence of the polymer body 20 to the tissue 98.

Retraction forces of up to 10 N and higher can be achieved without detachment of the muco-adhesive element 12 from the tissue 98 as has been shown in test experiments.

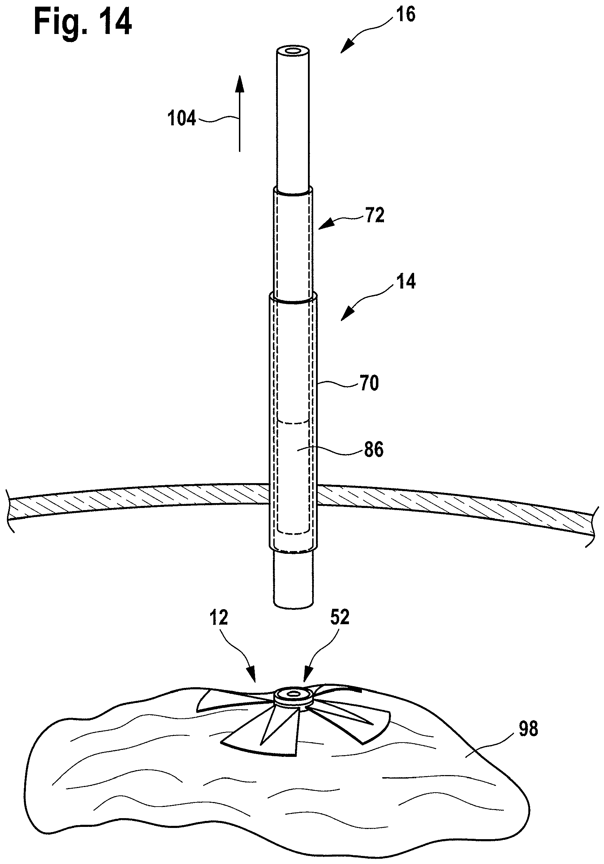

As shown in Fig. 14, it is also possible to detach the applicator 14 and manipulator 16 from the muco-adhesive element 12 at the surgical site, while the muco-adhesive element 12 further adheres to the tissue 98. This detachment of the applicator 14 and the manipulator 16 from the muco-adhesive element 12 is accomplished by retracting the manipulator 16 in proximal direction according to an arrow 104 without simultaneously retracting the slider 72. In this case, since the non-magnetic stop 76 of the slider 72 prevents the muco-adhesive element 12 from being drawn into the slider 72, retraction of the manipulator 16 alone has the effect that the magnetic attractive forces between the magnetic tip 86 and the magnetically acting portion 52 of the muco-adhesive element 12 are overcome.

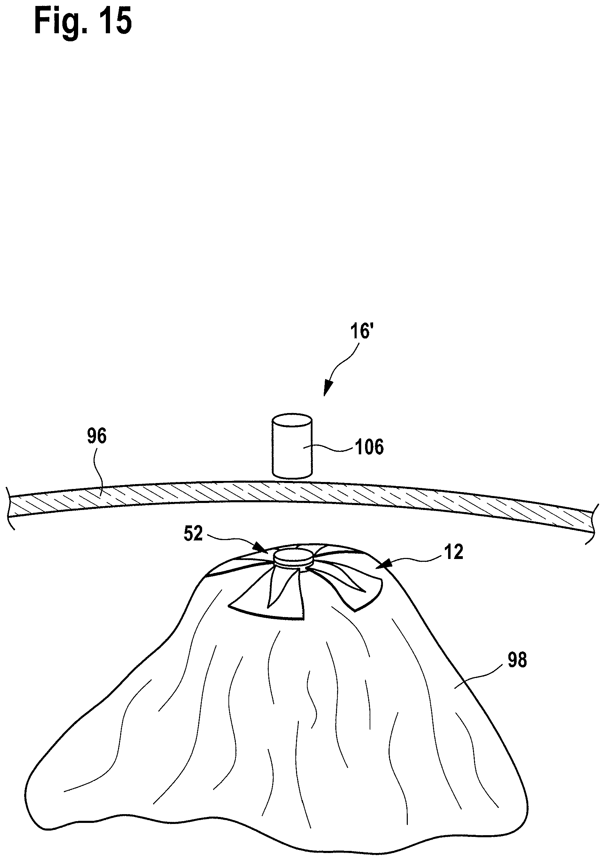

Fig. 15 shows another embodiment not forming part of the invention in which the applicator 14 and the manipulator 16' are not combined in one instrument, but are configured as separate instruments. In this case, the applicator 14 can only comprise the outer tube 70 and the slider 72 which in this case can be configured as a rod.

Differently from the manipulator 16 in the previous embodiment, the manipulator 16' is configured for extra-corporal remote magnetic coupling to the at least one magnetically acting portion 52 of the muco-adhesive element 12 when adhering to the tissue or organ 98, wherein the manipulator 16' acts through the body surface 96. In this case, the manipulator 16' can be configured as an external magnet or magnet system 106 which produces a sufficiently strong magnetic attraction force for remotely magnetically attracting the magnetically active portion 52 of the muco-adhesive element 12.

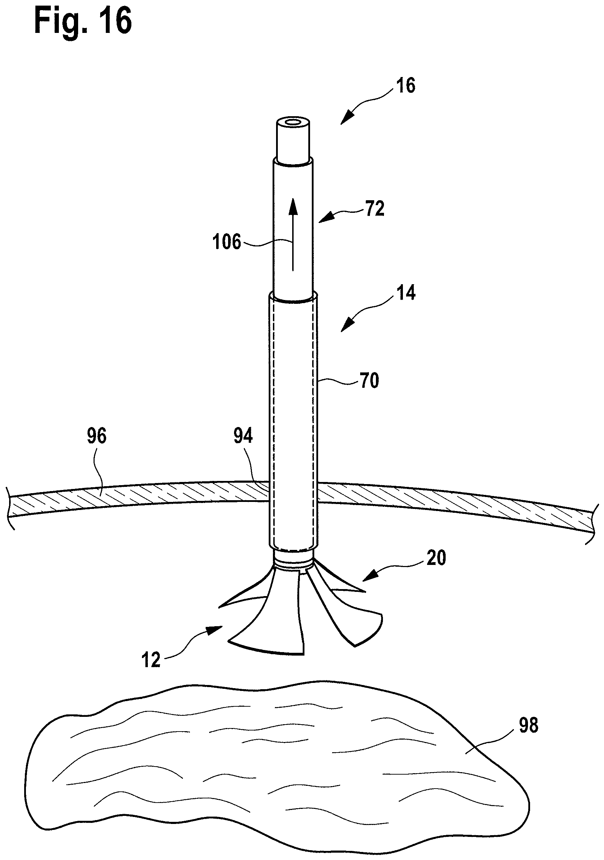

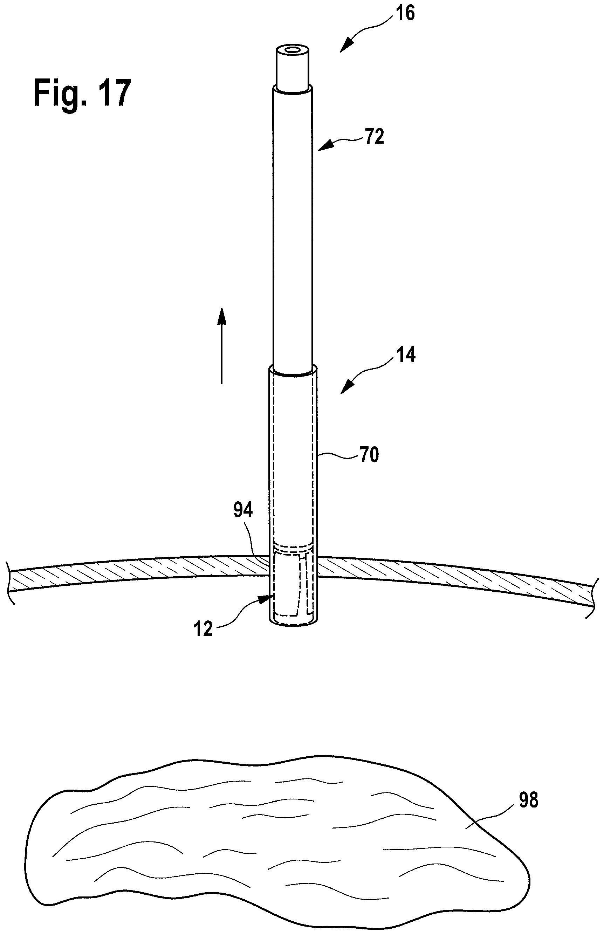

Referring back to the previous embodiment, Fig. 16 shows the use of the applicator 14 and the manipulator 16 for removing the muco-adhesive element 12 from the tissue 98 and withdrawal of the muco-adhesive element 12 through the minimal access port 94 in the body surface 96. First, starting from a state as shown in Fig. 12 (after the retraction procedure according to Fig. 13 has been finished), polymer body 20 has to be detached from the tissue 98 in a controlled manner. Controlled detachment of the polymer body 20 from the tissue 98 can be accomplished by delivering so much fluid (for example through the above-mentioned through holes 90 and 88 of the manipulator 16 and the through hole 80 of the non-magnetic stop 76 and the through hole 66 in the further magnet 58 and the through hole 60 in the polymer body 20) that the adherence of the polymer body 20 to the tissue 98 is significantly reduced by "over-moistening". "Over-moistening" results in a substantial weakening of the adherence of the polymer body 20 to the tissue 98 such that the muco-adhesive element 12 can be lifted from the tissue 98 without thereby retracting the latter. Next, withdrawal of the muco-adhesive element 12 from the tissue 98 is accomplished by retracting the slider 72 together with the manipulator 16 in proximal direction according to an arrow 106 in the same way as described with reference to Fig. 8, 9 and 10. The muco-adhesive element 12 is drawn into the outer tube 70 of the applicator 14 as shown in Fig. 17. Now, the outer tube 70 together with the slider 72 and the manipulator 16 and the muco-adhesive element 12 received in the outer tube 70 can be withdrawn from the minimal access port 94.

While in the embodiments described before the medical instrument kit 10 only has a single muco-adhesive element 12, it is also possible that the kit 10 comprises a plurality of muco-adhesive elements 12. For example, the medical instrument kit 10 can be provided with three or more muco-adhesive elements 12 which can be sequentially deployed and adhered to different areas of the target tissue, for example the target tissue 98 in Fig. 11. When using the external manipulator 16' as shown in Fig. 15, the external magnet or magnet system 106 can be much larger than shown in Fig. 15 in order to act in extra-corporal remote magnetic coupling with the plurality of adhered muco-adhesive elements 12, and the target organ/tissue 98 is retracted towards the abdominal wall 96 where the external magnet or magnet system 106 is located.

However, it is also possible to manipulate the plurality of muco-adhesive elements 12 adhering to the surface of the tissue 98 by using an internal manipulator introduced through the abdominal wall 96. For example, if four muco-adhesive elements 12 are used, they are sequentially deployed, for example via the same port (which can be smaller in size) and adhered in different areas of the target tissue/organ 98. Preferably, the four muco-adhesive elements 12 could be placed in a line. This is to facilitate following contact retraction, using a separate retraction probe having a magnet or magnet arrangement at the distal end which magnet or magnet arrangement has an elongated shape such that it can retract the four muco-adhesive elements 12 simultaneously.

## Claims

1. A medical instrument kit for manipulating, in particular retracting tissue or an organ (98) in the human or animal body, comprising:
a muco-adhesive element (12) having a flat-spread muco-adhesive polymer body (20) configured to adhere to the surface of the tissue or the organ (98), wherein the muco-adhesive element (12) further has at least one magnetically acting portion (52),
an applicator (14) configured to receive the muco-adhesive element (12) and to introduce the muco-adhesive element (12) into the body and for placing the muco-adhesive element (12) on the tissue or organ (98) to be manipulated, wherein the applicator (14) is configured to be introduced via a minimal access port (94) in the body surface (96) into the body, and
a manipulator (16) configured to magnetically couple to the at least one magnetically acting portion (52) of the muco-adhesive element (12), so that the tissue or organ (98) to which the muco-adhesive element (12) adheres can be manipulated through magnetic coupling between the manipulator (16) and the at least one magnetically acting portion (52) of the muco-adhesive element (12),
wherein the applicator (14) and the manipulator (16) are combined as one instrument (18).

2. The medical instrument kit of claim 1, wherein the muco-adhesive polymer body (20) is flexible so that it is foldable and/or can be rolled or wrapped for insertion into the applicator (14).

3. The medical instrument kit of claim 1 or 2, wherein the polymer body (20) is a thin film preferably having a thickness of less than 1 mm.

4. The medical instrument kit of any one of claims 1 through 3, wherein the muco-adhesive polymer body (20) has a plurality of strips (22, 24, 26, 28), wherein first longitudinal ends (29, 30, 32, 34) of the strips (22, 24, 26, 28) are connected with one another to form a central portion of the polymer body (20), and wherein second longitudinal ends (36, 38, 40, 42) of the strips (22, 24, 26, 28) opposite to the first longitudinal ends (29, 30, 32, 34) are free ends, wherein the strips (22, 24, 26, 28) extend in radial direction from the central portion.

5. The medical instrument kit of any one of claims 1 through 4, wherein the polymer body (20) has at least partially a stiffening layer (44, 46, 48, 50) for increasing the mechanical strength the polymer body (20).

6. The medical instrument kit of any one of claims 1 through 5, wherein the at least one magnetically acting portion (52) of the muco-adhesive element (12) has at least one magnet (54, 56) fixed to the polymer body (20).

7. The medical instrument kit of claim 6, wherein the at least one magnet (54, 56) comprises two magnets (54, 56), wherein the polymer body (20) is sandwiched between the two magnets (54, 56).

8. The medical instrument kit of claim 4 and 7, wherein the two magnets (54, 56) connect the first ends (29, 30, 32, 34) of the strips (22, 24, 26, 28) to one another by a clamping force produced by mutual magnetic attraction between the two magnets (54, 56).

9. The medical instrument kit of claim 7 or 8, wherein the two magnets (54, 56) are ring magnets defining a through hole (60) for receiving a further magnet (58).

10. The medical instrument kit of any one of claims 1 through 9, wherein the applicator (14) has an elongated outer tube (70) for receiving the muco-adhesive element (12), and an elongated slider (72) on the outer tube (70).

11. The medical instrument kit of claim 10, wherein the slider (72) is a second tube (74), and wherein the manipulator (16) is configured as a probe (82) adapted to slide in the second tube (74) and having a distal magnetic tip (86) for magnetic coupling to the at least one magnetically acting portion (52) of the muco-adhesive element (12).

12. The medical instrument kit of claim 11, wherein the second tube (74) has a distal non-magnetic stop (76).

13. The medical instrument kit of claim 9 and/or 11 and/or 12, wherein the muco-adhesive element (12) and/or the further magnet (58) and/or the probe (82) and/or the non-magnetic stop (76) has/have a through hole (60, 66, 88, 90, 80) extending in longitudinal direction for delivering a fluid for moistening the tissue or organ.

## Patentansprüche

1. Medizinisches Instrument-Kit zum Manipulieren, insbesondere Zurückziehen von Gewebe oder eines Organs (98) im menschlichen oder tierischen Körper, mit:
einem schleimhaftenden Element (12), das einen flach ausgebreiteten schleimhaftenden Polymerkörper (20) aufweist, der dazu ausgebildet ist, an der Oberfläche des Gewebes oder des Organs (98) zu haften, wobei das schleimhaftende Element (12) weiterhin zumindest einen magnetisch wirkenden Abschnitt (52) aufweist,
einem Applikator (14), der dazu ausgebildet ist, das schleimhaftende Element (12) aufzunehmen und das schleimhaftende Element (12) in den Körper einzuführen und zum Platzieren des schleimhaftenden Elements (12) auf dem zu manipulierenden Gewebe oder Organ (98), wobei der Applikator (14) dazu ausgebildet ist, über eine minimale Zugangsöffnung (94) in die Körperoberfläche (96) in den Körper hinein eingeführt zu werden, und
einem Manipulator (16), der dazu ausgebildet ist, magnetisch an den zumindest einen magnetisch wirkenden Abschnitt (52) des schleimhaftenden Elements (12) anzukoppeln, so dass das Gewebe oder Organ (98), an dem das schleimhaftende Element (12) haftet, durch magnetische Kopplung zwischen dem Manipulator (16) und dem zumindest einen magnetisch wirkenden Abschnitt (52) des schleimhaftenden Elements (12) manipuliert werden kann, wobei der Applikator (14) und der Manipulator (16) zu einem Instrument (18) kombiniert sind.

2. Medizinisches Instrument-Kit nach Anspruch 1, wobei der schleimhaftende Polymerkörper (20) flexibel ist, so dass er zum Einsetzen in den Applikator (14) faltbar ist und/oder gerollt oder gewickelt werden kann.

3. Medizinisches Instrument-Kit nach Anspruch 1 oder 2, wobei der Polymerkörper (20) ein dünner Film ist, der vorzugsweise eine Dicke von weniger als 1 mm aufweist.

4. Medizinisches Instrument-Kit nach einem der Ansprüche 1 bis 3, wobei der schleimhaftende Polymerkörper (20) eine Mehrzahl von Streifen (22, 24, 26, 28) aufweist, wobei erste Längsenden (29, 30, 32, 34) der Streifen (22, 24, 26, 28) miteinander verbunden sind, um einen zentralen Abschnitt des Polymerkörpers (20) zu bilden, und wobei zweite Längsenden (36, 38, 40, 42) der Streifen (22, 24, 26, 28), die zu den ersten Längsenden (29, 30, 32, 34) entgegengesetzt sind, freie Enden sind, wobei die Streifen (22, 24, 26, 28) sich in radialer Richtung von dem zentralen Abschnitt aus erstrecken.

5. Medizinisches Instrument-Kit nach einem der Ansprüche 1 bis 4, wobei der Polymerkörper (20) zumindest teilweise eine Versteifungsschicht (44, 46, 48, 50) zum Erhöhen der mechanischen Stärke des Polymerkörpers (20) aufweist.

6. Medizinisches Instrument-Kit nach einem der Ansprüche 1 bis 5, wobei der zumindest eine magnetisch wirkende Abschnitt (52) des schleimhaftenden Elements (12) zumindest einen Magneten (54, 56) aufweist, der an dem Polymerkörper (20) festgelegt ist.

7. Medizinisches Instrument-Kit nach Anspruch 6, wobei der zumindest eine Magnet (54, 56) zwei Magnete (54, 56) aufweist, wobei der Polymerkörper (20) zwischen den beiden Magneten (54, 56) in Sandwich-Anordnung angeordnet ist.

8. Medizinisches Instrument-Kit nach Anspruch 4 und 7, wobei die beiden Magnete (54, 56) die ersten Enden (29, 30, 32, 34) der Streifen (22, 24, 26, 28) durch eine Klemmkraft miteinander verbinden, die durch wechselseitige magnetische Anziehung zwischen den beiden Magneten (54, 56) erzeugt ist.

9. Medizinisches Instrument-Kit nach Anspruch 7 oder 8, wobei die beiden Magneten (54, 56) Ringmagnete sind, die ein Durchgangsloch (60) zum Aufnehmen eines weiteren Magneten (58) definieren.

10. Medizinisches Instrument-Kit nach einem der Ansprüche 1 bis 9, wobei der Applikator (14) ein langerstrecktes Außenrohr (70) zum Aufnehmen des schleimhaftenden Elements (12) und einen langerstreckten Gleiter (72) an dem Außenrohr (70) aufweist.

11. Medizinisches Instrument-Kit nach Anspruch 10, wobei der Gleiter (72) ein zweites Rohr (74) ist, und wobei der Manipulator (16) als Sonde (82) ausgebildet ist, die dazu ausgelegt ist, in dem zweiten Rohr (74) zu gleiten und eine distale magnetische Spitze (86) zum magnetischen Ankoppeln an den zumindest einen magnetisch wirkenden Abschnitt (52) des schleimhaftenden Elements (12) aufweist.

12. Medizinisches Instrument-Kit nach Anspruch 11, wobei das zweite Rohr (74) einen distalen nicht-magnetischen Anschlag (76) aufweist.

13. Medizinisches Instrument-Kit nach Anspruch 9 und/oder 11 und/oder 12, wobei das schleimhaftende Element (12) und/oder der weitere Magnet (58) und/oder die Sonde (82) und/oder der nicht-magnetische Anschlag (76) ein Durchgangsloch (60, 66, 88, 90, 80) aufweist/aufweisen, das sich in Längsrichtung zum Zuführen eines Fluides zum Anfeuchten des Gewebes oder Organs erstreckt.

## Revendications

1. Kit d'instruments médicaux pour manipuler, en particulier rétracter un tissu ou un organe (98) dans le corps humain ou animal, comprenant :
un élément muco-adhésif (12) ayant un corps en polymère muco-adhésif à plat (20) configuré pour adhérer à la surface du tissu ou de l'organe (98), où l'élément muco-adhésif (12) a en outre au moins une partie à action magnétique (52),
un applicateur (14) configuré pour recevoir l'élément muco-adhésif (12) et pour introduire l'élément muco-adhésif (12) dans le corps et pour placer l'élément muco-adhésif (12) sur le tissu ou l'organe (98) à manipuler, où l'applicateur (14) est configuré pour être introduit par un orifice d'accès minimal (94) dans la surface corporelle (96) dans le corps, et
un manipulateur (16) configuré pour se coupler magnétiquement à l'au moins une partie à action magnétique (52) de l'élément muco-adhésif (12), de sorte que le tissu ou l'organe (98) auquel adhère l'élément muco-adhésif (12) puisse être manipulé par couplage magnétique entre le manipulateur (16) et l'au moins une partie à action magnétique (52) de l'élément muco-adhésif (12), où l'applicateur (14) et le manipulateur (16) sont combinés pour former un seul instrument (18).

2. Kit d'instruments médicaux de la revendication 1, dans lequel le corps en polymère muco-adhésif (20) est flexible de sorte qu'il soit pliable et/ou puisse être enroulé ou roulé pour être inséré dans l'applicateur (14).

3. Kit d'instruments médicaux de la revendication 1 ou 2, dans lequel le corps en polymère (20) est un film mince ayant de préférence une épaisseur inférieure à 1 mm.

4. Kit d'instruments médicaux de l'une quelconque des revendications 1 à 3, dans lequel le corps en polymère muco-adhésif (20) a une pluralité de bandes (22, 24, 26, 28), où des premières extrémités longitudinales (29, 30, 32, 34) des bandes (22, 24, 26, 28) sont reliées les unes aux autres pour former une partie centrale du corps en polymère (20), et où des deuxièmes extrémités longitudinales (36, 38, 40, 42) des bandes (22, 24, 26, 28) opposées aux premières extrémités longitudinales (29, 30, 32, 34) sont des extrémités libres, où les bandes (22, 24, 26, 28) s'étendent dans une direction radiale à partir de la partie centrale.

5. Kit d'instruments médicaux de l'une quelconque des revendications 1 à 4, dans lequel le corps en polymère (20) a au moins partiellement une couche de raidissement (44, 46, 48, 50) pour augmenter la résistance mécanique du corps en polymère (20).

6. Kit d'instruments médicaux de l'une quelconque des revendications 1 à 5, dans lequel l'au moins une partie à action magnétique (52) de l'élément muco-adhésif (12) a au moins un aimant (54, 56) fixé au corps en polymère (20).

7. Kit d'instruments médicaux de la revendication 6, dans lequel l'au moins un aimant (54, 56) comprend deux aimants (54, 56), où le corps en polymère (20) est pris en tenaille entre les deux aimants (54, 56).

8. Kit d'instruments médicaux des revendications 4 et 7, dans lequel les deux aimants (54, 56) relient les premières extrémités (29, 30, 32, 34) des bandes (22, 24, 26, 28) les unes aux autres par une force de serrage produite par attraction magnétique mutuelle entre les deux aimants (54, 56).

9. Kit d'instruments médicaux de la revendication 7 ou 8, dans lequel les deux aimants (54, 56) sont des aimants annulaires définissant un trou traversant (60) pour recevoir un aimant supplémentaire (58).

10. Kit d'instruments médicaux de l'une quelconque des revendications 1 à 9, dans lequel l'applicateur (14) a un tube externe allongé (70) pour recevoir l'élément muco-adhésif (12), et un coulisseau allongé (72) sur le tube externe (70).

11. Kit d'instruments médicaux de la revendication 10, dans lequel le coulisseau (72) est un deuxième tube (74), et dans lequel le manipulateur (16) est configuré comme une sonde (82) adaptée pour coulisser dans le deuxième tube (74) et ayant une pointe magnétique distale (86) pour un couplage magnétique à l'au moins une partie à action magnétique (52) de l'élément muco-adhésif (12).

12. Kit d'instruments médicaux de la revendication 11, dans lequel le deuxième tube (74) a une butée distale non magnétique (76).

13. Kit d'instruments médicaux de la revendication 9 et/ou 11 et/ou 12, dans lequel l'élément muco-adhésif (12) et/ou l'aimant supplémentaire (58) et/ou la sonde (82) et/ou la butée non magnétique (76) a/ont un trou traversant (60, 66, 88, 90, 80) s'étendant dans une direction longitudinale afin de distribuer un fluide pour humidifier le tissu ou l'organe.
